# EUROPEAN PATENT SPECIFICATION

(11) **EP 4 702 948 B1**
(45) Date of publication and mention of the grant of the patent: **07.10.2026**
(21) Application number: 24197641.4
(22) Date of filing: 30.08.2024
(51) Int. Cl.: A61C 8/00, A61B 17/16

(54) **DENTAL SELF-TAPPING IMPLANT AND SUITABLE MULTI-STEP DRILL**
SELBSTBOHRENDES DENTALIMPLANTAT UND DAFÜR GEEIGNETER MEHRSTUFIGER BOHRER
IMPLANT DENTAIRE AUTOTARAUDEUR ET FORET À PLUSIEURS ÉTAPES APPROPRIÉ

(43) Date of publication of application: 04.03.2026
(73) Proprietor: EDUINVENT LM S.R.L., 077165 Judet IIfov (RO)
(72) Inventor: Steigmann, Marius, 077165 Judetul Ilfov (RO)
(74) Representative: Vasilescu, Raluca

(56) References cited:
- EP-A1- 3 398 553
- EP-A1- 4 324 424

## Description

### Field of the invention

The present invention refers to endosseous implants. In particular, the invention relates to a dental self-tapping device and to a multi-step drill suitable for the dental self-tapping device.

### Background of the invention

It is noticeable the desire of the implant industry to produce implants that cause less damage in the tissue of the patients while being are less difficult to be placed by the physicians.

Causing less damage and easing the implanting process can be carried out in multiple ways.

According to the prior art, recent self-tapping implants, alternatively called in this invention implants, typically comprise a body having a longitudinal axis along which a screwing axial direction is formed, the body having a body diameter. The body usually has a neck portion or dental connecting element to an abutment, a central portion and a distal portion with decreasing diameter.

The body has a core and threads provided thereon, and one or more flutes, alternatively called trenches, the flute(s)/trench(es) having various shapes among which a circular arc shape.

The one or more flutes are formed in the thread running spirally around the core, from the distal portion up to the neck portion or only on a part of the central portion and of the distal portion with decreasing diameter.

The flute typically forms an angle which is defined either between the axis of the flute and the longitudinal axis or between the axis of the flute and the tangent direction of the thread portion.

Said angle is forming the cutting edges of the implant.

Documents US2015/0297321, US2020/0337811 A1, EP22204148.5 and EP4324424A1 provide examples of self-tapping implants of the prior art.

### Disadvantages of prior art

The cited technical solutions disclosed above, while achieving partially the objective of easing the implanting process, are still away from the objective of reducing damage in the tissue of the patients.

Specifically, the nature and quality of alveolar bone and its variability among the patients is still not taken sufficiently into account as an important factor when designing the implants.

The inventor realized that tissue damage occurs during the implanting process because the implants are still not sufficiently adapted to the anatomy particulars of the jawbones.

Typically, the jawbones have two layers:
- an outer, denser layer, called the cortical tissue of the jawbone, hereafter in short, the cortical tissue, and
- an inner, softer layer, called the spongiosa tissue of the jawbone, hereafter in short, the spongiosa tissue.

The cortical tissue is the most mineralized and hardest layer, having a greater mechanical resistance and greater density than the spongiosa tissue.

There are different grades of hardness of the jawbones depending on the position of the missing tooth/teeth on the upper or the lower jawbone of the patient, and there are different grades of hardness of the same jawbone among different patients.

The usual dimensions of the cortical tissue of the jawbone and of the spongiosa tissue of the jawbone are known from medical literature.

Many implants of prior art have a high probability to be blocked during the implanting process, particularly when screwing in the cortical tissue, because of the need to increase the torque values as compared with the values of the torque needed to screw in the spongiosa tissue.

Increasing torque values usually leads to excessive torque values, whose immediate consequence is over-compression of the cortical tissue, said over-compression leading to compression necrosis of the cortical tissue surrounding the osteotomy (osteotomy = the orifice in the jawbone of the patient where the implant will be implanted). The necrosis, in turn, negatively affects both the primary stability of the implant - namely the mechanical engagement of the implant in its surrounding bone, and the secondary stability- namely the successful osseointegration over time.

In addition, wider-diameter implants in the portions of the jawbones where the density of the cortical tissues is highest, have a supplementary reason to cause compression necrosis of the cortical tissue surrounding the osteotomy, because the wider the diameter of the implant, the greater is the friction force.

Yet another disadvantage is that, as the conventional drills are reusable, they must be sterilized. Multiple sterilization causes loss of sharpening of the conventional drills, reason for which the conventional drills need to be re-sharpened from time to time. Experience shows that in many places, the drills are not sterilized and/or re-sharpened properly which obviously contribute to increasing the probability of the afore-mentioned compression necrosis of the cortical tissue.

There are two current solutions used in order to avoid compression necrosis, given that the cortical tissue is denser:
i) either using an implant having the diameter of the body close to the proximal portion smaller than the diameter of the body of the remaining section - case in which the risk of compression necrosis is diminished, but the primary stability of the implant is not satisfactory, because of the existence of extra-space between the osteotomy and the implant prevents fixing the implant in the dense part of the bone - that is the cortical tissue, the implant being essentially fixed in the spongiosa tissue which is softer, or
ii) using a conventional reusable tap drill as an additional tool - case in which the risk of producing compression necrosis still remains, because of the friction forces and the lateral compression during drilling, as it is difficult to match the dimensions of the conventional tap drill to the dimensions of the implant and for the general disadvantages of the reusable drills mentioned above.

### Problem to be solved by the invention

The inventor aimed to conceive a new dental self-tapping device better adapted to the anatomy particulars of the osteotomy, which improves the primary stability, such that the risk of over-compression necrosis be significantly lowered, particularly the over-compression necrosis of the cortical tissue.

Specifically, the technical problem to be solved by the invention is to adapt the geometry of the self-tapping implant:
- to the variation of the hardness between the cortical tissue and the spongiosa tissue, and
- to the diameter of the body of the self-tapping implant,
such that to reduce significantly the risk of over-compression of the cortical tissue during the implanting process.

### Summary of the invention

In order to overcome the disadvantages of prior art, in a first aspect of the invention it is presented a dental self-tapping implant comprising a body having a longitudinal body axis and a length, the body having a proximal neck portion, a proximal-central straight portion of substantially constant body diameter and a distal conical portion of decreasing diameter, the body comprising a core with a thread provided thereon, and a flute of circular arc shape formed in the thread, the flute runs helicoidally around the core, the flute having a longitudinal flute axis,

The body has two segments:
- a cortical segment next to the proximal neck portion,
- a spongiosa segment extending the cortical segment, the spongiosa segment including the distal conical portion,
the sum of the cortical segment and the spongiosa segment being equal to the sum of the proximal-central straight portion and the distal conical portion,

The flute is configured to run helicoidally around the core until it reaches the neck portion, so that the longitudinal flute axis forms a first angle with the longitudinal body axis along the cortical segment, and forms a second angle with the longitudinal body axis along the spongiosa segment,

The first angle is larger than the second angle.

The value of the first angle is directly proportional to the body diameter with a proportionality parameter within the interval 11 to 18, preferably within the interval 13 to 15.

In a second aspect of the invention, it is presented set comprising a dental self-tapping implant and a multi-step drill configured for the dental self-tapping implant.

The multi-step drill has four steps along the spongiosa segment, each step having a drill diameter and a step height.

The drill diameters increase from the portion of the multi-step drill corresponding to the distal conical portion to the proximal-central straight portion.

The body diameter is greater than the drill diameters at each step height.

The step heights along the spongiosa segment have respective predetermined values irrespective of the body diameter.

For various dental self-tapping implants of the invention having different body diameters, the drill diameters increase with the respective body diameter.

In a third aspect of the invention, it is presented a collection comprising at least three sets, each set comprising a dental self-tapping implant and a multi-step drill 100 configured for the dental self-tapping implant.

The diameter of each of the at least three dental self-tapping implants of the collection is selected from a list of diameters that forms an increasing series with a step interval of at least 0.2 mm, preferably with a step interval of 0.3 to 0.5 mm, more preferably with a step interval of 0.4 mm, and the cores of the dental self-tapping implants are substantially identical for all the selected diameters from the list.

### Advantages

The self-tapping dental implant of the invention has the following advantages:
- reduces significantly the risk of over-compression of the cortical tissue during the implanting process and eases the implanting process,

- as result of reducing the risk of over-compression and of easing the implanting process, the implant of the invention achieves the improvement of the primary stability of the implant and the osseointegration in both types of tissues - the cortical tissue and the spongiosa tissue, which is the ultimate goal of the invention.

The set of the invention comprising the dental self-tapping implant and the multi-step drill configured for the dental self-tapping implant has the advantages of the dental self-tapping implant and the following further advantages:
- further reduces the risk of over-compression of the cortical tissue during the implanting process because it eliminates part of the cause of over-compression when using conventional drill(s), as the dental self-tapping implant is better anchored in the osteotomy,
- the implanting process is more precise,
- further eases the implanting process because it reduces the time and complexity to the surgical procedure, since it is no longer necessary to use multiple drills,
- as a result of further reducing the risk of over-compression and further easing the implanting process, the set of the invention further achieves the improvement of the primary stability of the implant and the osseointegration in both types of tissues,
- for the embodiments when the multi-step drill of the invention is disposed at the end of the implanting process, the set of the invention provides safer hygienic conditions for the patients, because the multi-step drill of the invention is always properly sharpened and there is no need to sterilize it.

### List of drawings

Further special features and advantages of the present invention can be taken from the following description of an advantageous embodiment by way of the accompanying drawings:

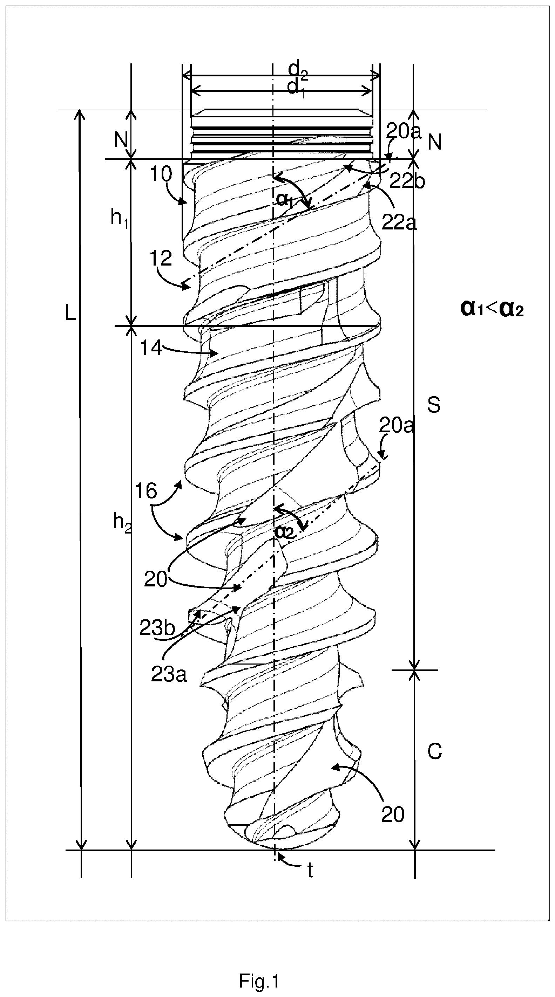

Fig.1 pictures the dental self-tapping implant according to the invention showing the difference between the two inclinations of the angles of the flute, specifically that the angle **α₁** along the cortical segment **h₁** is larger than the second angle **α₂** along the spongiosa segment **h₂**: **α₁** > **α₂**.

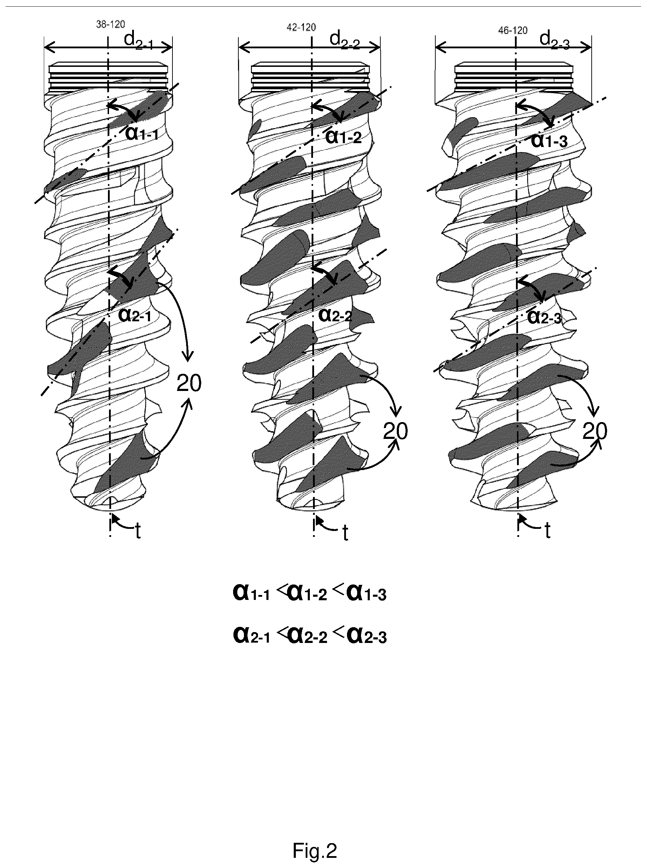

Fig.2 pictures the dental self-tapping implant according to the invention as depicted in Fig. 1, in three different examples of realization for various body diameters **d₂**, the length L remaining constant L= 120mm: **d₂₋₁**= 3.8 mm; **d₂₋₂**= 4.2 mm; and **d₂₋₃**= 4.6mm, showing how the angles **α₁** and **α₂** vary with the body diameter **d₂**.

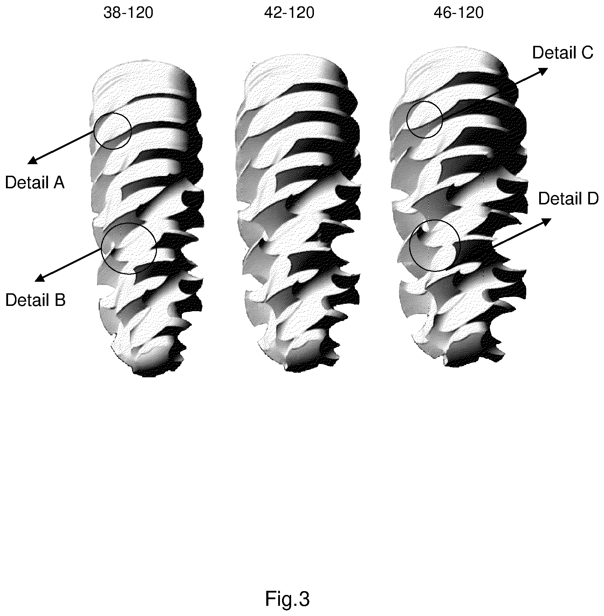

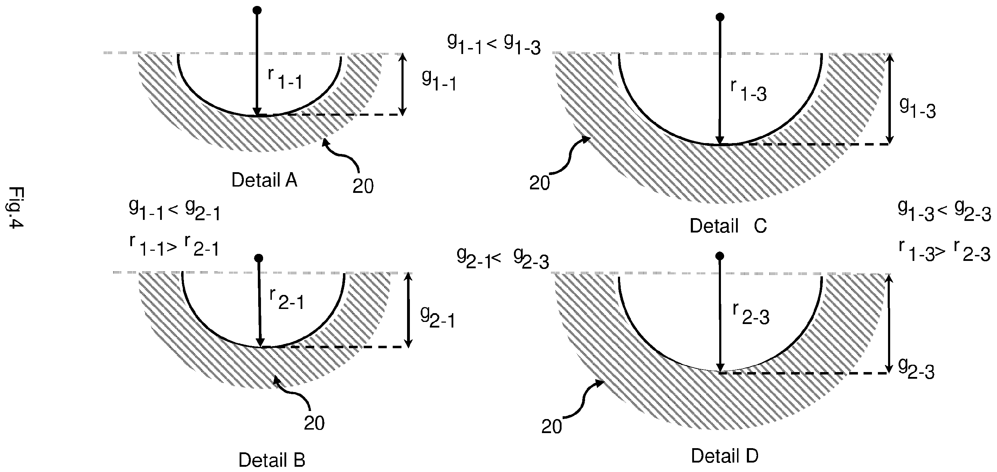

Fig.3 shows a perspective view of the three different examples of realization for various body diameters **d₂** from Fig.2, and indicates details A, B, from the first embodiment of Fig.2 having **d₂₋₁**= 3.8 mm and, respectively details C, D from the third embodiment of Fig.2 having **d₂₋₃**= 4.6mm, said details A,B,C,D showing the corresponding depths **g₁, g₂** and the corresponding radii **r₁, r₂,** said details making the object of Fig.4.
Fig. 4 shows a schematic cross-view of the flute of the first embodiment of Fig. 2, having the body diameter **d₂₋₁=** 3.8 mm, and of the third embodiment of Fig. 2, having the body diameter **d₂₋₃=** 4.6mm showing the four details A, B, C, D indicated in Fig. 3, depicting schematically the relationship of the respective depths and radii of the flute corresponding to the cortical segment **h₁** and, respectively, the spongiosa segment **h₂**.

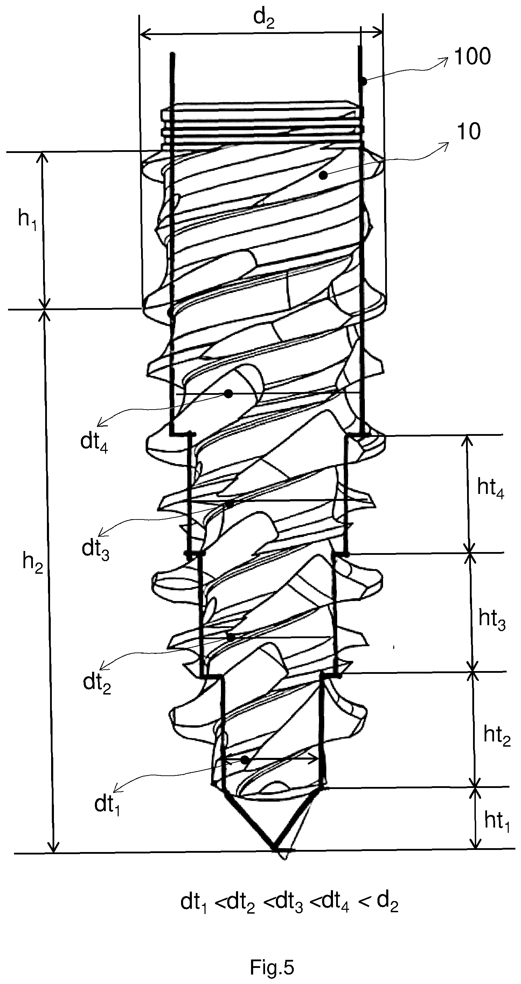

Fig. 5 shows the steps of the multi-step drill of the set of the invention, said steps conceived to be adapted to the dental self-tapping implant according to the invention of Fig.1. It shows a schematic, imaginary superposition of the multi-step drill to the dental self-tapping implant for the ease of understanding of the adaptations of the multi-step drill to the dental self-tapping implant.

### Detailed description and example of realization

In a first aspect of the invention, with reference to Fig 1, Fig. 2, it is presented a dental self-tapping implant 10 comprising a body 12 having a longitudinal body axis **t** and a length L, the body 12 having a proximal neck portion N, a proximal-central straight portion S of substantially constant body diameter d₂ and a distal conical portion C of decreasing diameter, the body 12 comprising a core 14 with a thread 16 provided thereon, and a flute 20 of circular arc shape formed in the thread 16, the flute 20 runs helicoidally around the core 14, the flute 20 having a longitudinal flute axis 20a.

The dental self-tapping implant 10 is configured to be implanted in patient's lower or upper jawbone, which has the two afore-mentioned types of tissues:
- the cortical tissue, and
- the spongiosa tissue.

It is known that the self-tapping implants are manufactured with various body diameters **d₂**, whose values are known by the skilled person. Each body diameter **d₂** is better fit for specific position of the missing tooth/teeth where the self-tapping implant will be placed.

The following non-limiting examples of various body diameters **d₂** will be used in the invention for exemplification- with reference to Fig. 2:
**d₂₋₁** = 3.8 mm; **d₂₋₂** = 4.2 mm; **d₂₋₃** =4.6 mm

It is also known that the self-tapping implants are manufactured with various lengths L of the implants, where said length L is the sum of proximal neck portion N, the proximal-central straight portion S and the distal conical portion C: [Eq. 1] $L = C + S + N$

The following non-limiting example of lengths L of the implants will be used in the invention for exemplification- with reference to Fig. 1 and Fig. 2: L = 120 mm.

The teaching of the invention is applicable to any body diameter **d₂**, as well as to any length L that are different from the ones exemplified above.

The principle underlying the invention is the following: the inventor found that, in order to reduce significantly the risk of over-compression of the cortical tissue during the implanting process - that is to solve the technical problem of the invention, the torque when screwing the implant should preferably be substantially constant irrespective of the body diameter **d₂**, and irrespective of the bone hardness.

The torque should preferably be maintained in the range between 25 Ncm to 70 Ncm, more preferably 35 Ncm and 50 Ncm.

All the features of the invention as claimed are designed to contribute synergically to maintaining the torque substantially constant.

The features of the invention are described below.

The body 12 has two segments:
a cortical segment **h₁** next to the proximal neck portion N,
a spongiosa segment **h₂** extending the cortical segment **h₁**, the spongiosa segment **h₂** including the distal conical portion C,

Mathematically speaking, the sum of the cortical segment **h₁** and the spongiosa segment **h₂** is equal to the sum of the proximal-central straight portion S and the distal conical portion C: [Eq. 2] $C + S = {h}_{1} + {h}_{2}$

The respective lengths of the cortical segment **h₁** and of the spongiosa segment **h₂**, measured along the longitudinal body axis **t** are such that, when the dental implant is in place in patient's lower or upper jawbone, the cortical segment **h₁** essentially corresponds to the cortical tissue, while the spongiosa segment **h₂** essentially corresponds to the spongiosa tissue.

The length of the cortical segment **h₁** is substantially constant for all lengths L of the implants, as it depends on the dimension of the cortical tissue of the patient which in its turn is substantially constant for a certain place of the lower or upper jawbone of the patient, and, as such, known in the literature.

One non-limiting example of calculating the length of the cortical segment **h₁** by using an average of the dimension of the cortical tissues from the literature.

The length of the spongiosa segment **h₂** has different values for different lengths L of the implants.

With reference to Fig. 1 and Fig. 2, the flute 20 is configured to run helicoidally around the core 14 until it reaches the neck portion N, so that the longitudinal flute axis 20 forms a first angle **α₁** with the longitudinal body axis **t** along the cortical segment **h₁**, and forms a second angle **α₂** with the longitudinal body axis **t** along the spongiosa segment **h₂**.

Unlike the prior art - where the angle of the flute with the longitudinal body axis is essentially constant along the entire length of the flute, in the invention, the division of the sum of the proximal-central straight portion S and the distal conical portion C into the two segments - the cortical segment **h₁** and the spongiosa segment **h₂** allows the creation of the two different angles: the first angle **α₁** and the second angle **α₂**.

Unlike the prior art, the flute 20 of the invention - having the first angle α₁ and the second angle α₂, is configured to run helicoidally around the core 14 until it reaches the neck portion N.

The relationship between the first angle **α₁** and the second angle **α₂** is as follows: the first angle **α₁** is larger than the second angle **α₂** [Eq. 3] ${α}_{1} > {α}_{2} ,$

As seen in Fig. 2, the value of the first angle **α₁** is directly proportional to the body diameter d₂ with a proportionality parameter k. [Eq. 4] ${α}_{1} = {d}_{2} ∗ k$

The value of the proportionality parameter k varies within the interval [11;18], preferably within the interval [13,15].

A non-limiting example is presented below:
**d₂₋₁** = 3.8 mm; **d₂₋₂** = 4.2 mm; **d₂₋₃** = 4.6 mm, **k =** 14
α **₁₋₁** = 3.8*14 = 53.2 °, α **₁₋₂** =4.2 *14= 58.8°, α **₁₋₃** =4.6*14= 64.4°

The advantages of the dental self-tapping implant 10 according to the invention stem from the following line of reasoning:
- the necrosis, particularly the necrosis of the cortical tissue is caused by over-compression of said cortical tissue,
- the over-compression when using prior art implants is caused by increased torque when screwing, excessive torque being frequent when screwing in the cortical tissue, in particular for implants of larger body diameter **d₂**,
- in order to reduce over-compression, the torque should preferably be substantially constant irrespective of the body diameter **d₂**, and irrespective of the bone hardness, and should preferably be maintained in the range between 25 Ncm to 70 Ncm, more preferably 35 Ncm and 50 Ncm,
- the objective of keeping the torque substantially constant along the longitudinal body axis t is achieved as a synergic effect of the geometrical characteristics of the dental implant 10, as claimed:
   - Given that the longitudinal flute axis 20 forms the first angle α₁ and, respectively the second angle α₂ with the longitudinal body axis t, the two angles respectively corresponding to the cortical segment **h₁**, and the spongiosa segment **h₂**, there are two sets of cutting edges, instead of one as in the prior art, namely: the cutting edges along the cortical segment **h₁** 22a, 22b and, respectively the cutting edges along the spongiosa segment **h₂** 23a, 23b.
   - Because the first angle α₁ is larger than the second angle α₂, the effect is an increased cutting power of the cutting edges along the cortical segment **h₁**, 22a, 22b, as compared to the cutting power of the cutting edges along the spongiosa segment **h₂** 23a, 23b, said increased cutting power compensating the need to increase the torque needed according to prior art. In other words, the cutting edges along the cortical segment **h₁**, 22a, 22b are sharper than the cutting edges along the spongiosa segment **h₂** 23a, 23b.
   - Because the first angle **α₁** is determined direct proportionally to the body diameter **d₂**, this means that larger values of the first angle α₁ are determined for wider body diameters **d₂**, whose effect is an increased cutting power of the cutting edges along the cortical segment **h₁** 22a, 22b as compared with the cutting power of the of the cutting edges along the cortical segment **h₁** 22a, 22b for narrower body diameters d₂, said increased cutting power compensating the need to increase the torque of prior art. In other words, the larger the diameter d₂ is, the sharper the cutting edges along the cortical segment **h₁**, 22a, 22b. When increasing the diameter of the implant, not only the sharpness is increased but also the cutting surface due to flute angulation, as the skilled person shall understand.

The fact that the flute 20 runs helicoidally around the core covering the full length of the threaded portion, combined with the fact that the longitudinal flute axis 20a forms said first angle **α₁** which is larger than said second angle **α₂**, makes useless the use of any conventional tap drill as additional tool when preparing the osteotomy, because the dental self-tapping implant 10 itself acts as a tap drill for both the cortical tissue and the spongiosa tissue, improving in this way the fixing capacity of the dental self-tapping implant 10 in both the cortical tissue and the spongiosa tissue. The fact that dental self-tapping implant 10 itself acts as a tap drill is more important for the cortical tissue because it eliminates the need to use conventional reusable tap drill(s) as an additional tool. By improving the fixing capacity of the implant, the primary stability is also improved. This is an additional advantage of improving the fixing capacity the dental self-tapping implant 10 of the invention, while reducing at the same time the risk of over-compression.

The inventor found that the objective of maintaining the torque substantially constant along the longitudinal body axis t of the implant irrespective of value of the body diameter **d₂** of the implant is better achieved by defining a dependency between the first angle **α₁** and the second angle **α₂**, because, by virtue of dependency on the first angle **α₁**, the second angle **α₂** will also vary direct proportionally to the body diameter **d₂**.

For this reason, in a preferred embodiment, with reference to Fig 1 and fig. 2, a first ratio q is defined between the first angle **α₁** and the second angle **α₂**, the first ratio q being in the interval [1.09; 1.15], preferably [1.115; 1.125], more preferably 1.12. [Eq. 5] $q = {α}_{1} / {α}_{2}$

The best results were achieved with the most preferred values **q=** 1.12.

Turning to the non-limiting examples, with reference to Fig 1 and Fig.2 and taking the most preferred value q= 1.12 one can find out the values of the second angle α₂:
α₂₋₁ =47.5°; α₂₋₂ =52.5°; α₂₋₃ =57.5°;

The inventor found that the objective of maintaining the torque substantially constant along the longitudinal body axis t of the implant irrespective of value of the body diameter **d₂** of the implant is further achieved by defining two depths of the flute 20.

With reference to Fig. 3, given that the longitudinal flute axis 20a forms the first angle **α₁** and, respectively the second angle **α₂** with the longitudinal body axis t, the two angles respectively corresponding to the cortical segment **h₁**, and the spongiosa segment **h₂**, there are two depths of the flute 20 instead of one as in the prior art:
Specifically, the flute 20 has a first depth **g₁** along the cortical segment **h₁**, and a second depth **g₂** along the spongiosa segment **h₂**.

The first depth **g₁** and the second depth **g₂** are measured in radial direction from the tangent to the body 12 in the respective section.

The first depth **g₁** is smaller than the second depth **g₂**. [Eq. 6] ${g}_{1} < {g}_{2}$

Because the first depth **g₁** is smaller than the second depth **g₂**, the cortical segment **h₁** overlaps partially with the abutment area which is one reason more for the first depth **g₁** to be smaller than the second depth **g₂**.

Both the first depth **g₁** and the second depth **g₂** increase with the body diameter **d₂**, further positively contributing to the better evacuation of the bone scraps when larger body diameters **d₂** are used as compared with the cases when smaller body diameters **d₂** are used.

Practically, there is a synergic effect between the variation of the two angles and the variation of the two depths corresponding to the cortical segment **h₁**, and, respectively the spongiosa segment **h₂** of the flute 20: when the angle increases - **α₁** > **α₂**, the depth decreases - **g₁** < **g₂**

The effect of the variation is a better adaptation of the implant to the anatomy particulars of the osteotomy, because the larger angle and the smaller depth corresponding to the cortical tissue increases the cutting power of the dental implant, while the smaller angle and the increased depth corresponding to the spongiosa tissue decreases the cutting power and increases the anchoring in the spongiosa tissue.

The relationship between the first depth **g₁** and the second depth **g₂** together with the dependency of the first depth **g₁** and the second depth **g₂** to the body diameter **d₂** does produce its positive effect on the objective of maintaining the torque substantially constant along the longitudinal body axis **t** of the implant irrespective of value of the body diameter d₂ of the implant irrespective of whether the first ratio **q** is used or not. However, the skilled person will understand that by using the first ratio **q** with its preferred value a better result than if no first ratio **q** is used.

The inventor found that the objective of maintaining the torque substantially constant along the longitudinal body axis **t** of the implant irrespective of value of the body diameter d₂ of the implant is further achieved by defining two radii of the flute 20.

With reference to Fig. 3, given that the longitudinal flute axis 20a forms the first angle **α₁** and, respectively the second angle **α₂** with the longitudinal body axis t, the two angles respectively corresponding to the cortical segment **h₁**, and the spongiosa segment **h₂**, there are two radii of the flute 20 instead of one as in the prior art:
Specifically, the circular arc shape of the flute 20 has a first radius **r₁** along the cortical segment **h₁**, and a second radius **r₂** along the spongiosa segment **h₂**.

The first radius **r₁** is larger than the second radius **r₂**. [Eq. 7] ${r}_{1} > {r}_{2}$

Because the first radius **r₁** is larger than the second radius **r₂**, the bone scraps are better evacuated than in prior art cases where there is only one depth of the flute, given that the volume of said bone scraps is higher in the spongiosa segment **h₂** than in the cortical segment **h₁**.

The relationship between the first radius **r₁** and the second radius **r₂** does produce its positive effect on the objective of maintaining the torque substantially constant along the longitudinal body axis **t** of the implant irrespective of value of the body diameter **d₂** of the implant irrespective of whether the first ratio **q** is used or not. However, the skilled person will understand that by using the first ratio **q** with its preferred value a better result than if no first ratio **q** is used.

Furthermore, by combining the feature of the relationship between the first depth **g₁** and the second depth **g₂** together with the dependency of the first depth **g₁** and the second depth **g₂** to the body diameter **d₂** with the feature of the relationship between the first radius **r₁** and the second radius r₂, increases the positive effect on the objective of maintaining the torque substantially constant along the longitudinal body axis **t** of the implant irrespective of value of the body diameter d₂ of the implant, because each of the features that are claimed has its specific contribution to the fine - tuning of the torque.

The more geometrical features claimed in the invention are used in the implant, according to the various embodiments and combination of embodiments described briefly above, better is for the fine-tunning of the torque and, thus, better the invention achieves to avoid significantly the risk of over-compression of the cortical tissue during the implanting process and, hence, the better the invention achieves the improvement of the primary stability of the implant and the osseointegration which is the ultimate goal.

In a second aspect of the invention, with reference to Fig. 5, it is presented a set comprising the dental self-tapping implant 10 of the invention together with a multi-step drill 100 configured for the dental self-tapping implant 10.

The principle behind the second aspect of the invention is to remedy the disadvantages discussed above in respect to the use of conventional drill(s).

The multi-step drill 100 is either reusable or disposable, the decision to use it or to dispose it being a business one, outside the scope of the invention. The configuration of the multi-step drill 100 is the same irrespective of whether it is reusable or disposable.

The material of confection of the multi-step drill 100 is different for the two afore-mentioned situations: when the multi-step drill 100 is reusable, the material must allow sharpening and sterilization for a number of times according to the applicable norms.

If the multi-step drill 100 of the invention is disposed at the end of the implanting process, the set of the invention provides safer conditions for the patients, because it eliminates the source of infection due to a possible wrong sterilization and/or inappropriate sharpening.

The multi-step drill 100 of the invention is destined to drill in the spongiosa tissue, because the issue of drilling in the cortical issue is solved by the fact that dental self-tapping implant 10 itself acts as a tap drill, which is particularly important for the cortical tissue because it eliminates the need to use conventional reusable tap drill(s) as an additional tool.

The role of the multi-step drill 100 of the invention is to improve the anchoring of the dental self-tapping implant 10 in said spongiosa tissue and by this, to contribute the set of the invention can further contribute to the fine tuning of the torque needed and, thus, to further resolve the problem of the invention.

The multi-step drill 100 has four steps along the spongiosa segment **h₂**, each step having a drill diameter **dtᵢ** and a step height **htᵢ**, where **i=** 1,2,3,4.

Fig. 5 shows a schematic, imaginary superposition of the multi-step drill to the dental self-tapping implant for the ease of understanding of the adaptations of the multi-step drill to the dental self-tapping implant, as the shape of the multi-step drill theoretically matches the shape of the osteotomy at least when it comes to the spongiosa tissue.

The specific adaptations of the multi-step drill 100 to the dental self-tapping implant 10 of the invention are the following:
- the drill diameters **dtᵢ** increase from the portion of the multi-step drill 100 corresponding to the distal conical portion C to the proximal-central straight portion S,
- the body diameter d₂ is greater than the drill diameters **dtᵢ** at each step height **htᵢ** where **i=** 1,2,3,4 and in that:
- the step heights **htᵢ** along the spongiosa segment **h₂** have respective predetermined values irrespective of the body diameter **d₂,**
- for various dental self-tapping implants of the invention having different body diameters **d₂** the drill diameters **dti** increase with the respective body diameter **d₂**.

As seen in Fig. 5:
- The shape of the first step is conical with the pointed top of the cone being adapted to the shape of the distal conical portion C, the first drill diameter **dt₁** being the one opposite the distal conical portion C. The first step height is **ht₁** and the first drill diameter is **dt₁,**
- The second step is adjacent to the first step towards the proximal-central straight portion S and has a substantially cylindrical shape. The second step height is **ht₂,** and the second drill diameter is **dt₂,**
- The third step is adjacent to the second step and has a third drill diameter **dt₃** and has a substantially cylindrical shape. The third step height is **ht₃**, and the third drill diameter is **dt₃**,
- The fourth step is adjacent to the third step and has a substantially cylindrical shape. The fourth step height is **ht₄,** and the fourth drill diameter **dt₄,**
- The increase of the drill diameters **dtᵢ** with the body diameter **d₂** is due to the conicity of the dental self-tapping implant 10 of the invention,
- The remaining portion of the multi-step drill 100 corresponds with the proximal-central straight portion S of the implant,
- The step heights **htᵢ** do not depend on the body diameter **d₂** because they do not depend on the conicity of the dental self-tapping implant 10 of the invention. Mathematically speaking: [Eq. 8] ${dt}_{1} < {dt}_{2} < {dt}_{3} < {dt}_{4} < {d}_{2}$

Non-limiting examples of values of the drill diameters **dtᵢ** for the three examples of various body diameters d₂ shown in Fig. 2:
For **d₂₋₁** = 3.8 mm: **dt₁₋₁=** 2mm; **dt₁₋₂**=2.2mm; **dt₁₋₃** =2.6mm; **dt₁₋₄**=3.1mm
For **d₂₋₂** = 4.2 mm: **dt₂₋₁=** 2mm; **dt₂₋₂**=2.2mm; **dt**_{**2**-3} =2.8mm; **dt₂₋₄**=3.3mm
For **d₂₋₃** =4.6 mm: **dt₃₋₁**= 2mm; **dt₃₋₂**=2.7mm; **dt₃₋₃** =3.0mm; **dt₃₋₄**=3.3mm

Non-limiting examples of values of the step heights **htᵢ** - for all body diameters d₂:
**ht₁=** 0.5mm; **ht₂**=2mm; **ht₃**=2mm; **ht₄**=2mm

By using the multi-step drill of the set of the invention when drilling in patient's jawbone, the drilling force is applied gradually as compared with the use of a conventional conical drill of prior art, which further reduces the risk of over-compression compared with the situation in which the drilling is carried out with the conventional drill(s) of prior art.

When the drilling force is applied during the implanting process, because the body diameter **d₂** is greater than the drill diameters **dtᵢ**, two phenomena occur:
- a lateral compression occurs in the spongiosa tissue, and
- a wedging effect is created between the osteotomy having the shape of the multi-step drill configured for the dental self-tapping implant, and said dental self-tapping implant

The two mentioned phenomena produce a synergic effect having as result a better anchoring of the thread of the dental self-tapping implant in the osteotomy compared with the situation in which the drilling is carried out with a conventional drill of prior art, which means a more precise implanting process.

Using the multi-step drill of the set of the invention **further** eases the implanting process because it reduces the time and complexity to the surgical procedure, since it is no longer necessary to use multiple drills.

For the embodiments when the multi-step drill of the invention is disposed at the end of the implanting process, the set of the invention provides safer hygienic conditions for the patients, because the multi-step drill of the invention is always properly sharpened and there is no need to sterilize it.

For all the reasons above, the use of the set comprising the dental self-tapping implant of the invention and the multi-step drill configured for the dental self-tapping implant provides **further** improvement of the primary stability of the implant and the osseointegration in both types of tissues as compared with the cases when the dental self-tapping implant of the invention is used together with a conventional drill.

In a third aspect of the invention, it is presented a collection comprising at least three sets, each set comprising a dental self-tapping implant and a multi-step drill 100 configured for the dental self-tapping implant.

The diameter **d₂** of each of the at least three dental self-tapping implants 10 is selected from a list of diameters that forms an increasing series with a step interval of at least 0.2 mm, preferably with a step interval of 0.3 to 0.5 mm, more preferably with a step interval of 0.4 mm, and the cores 14 of the dental self-tapping implants are substantially identical for all the selected diameters **d₂** from the list.

The multi-step drill 100 of the collection is either reusable or disposable.

The collection has the advantage of further easing the implanting process because it provides together the instruments that are needed for the implanting process: the implant and its corresponding multi-step drill. The possibility to select the diameter **d₂** from a list of diameters provides flexibility in creating the collection according to various needs.

All the examples regarding the body diameter correspond to the third aspect of the invention, with reference to Fig. 2, where the step interval is 0.4mm:
**d₂₋₁** = 3.8 mm; **d₂₋₂** = 4.2 mm; **d₂₋₃** =4.6 mm

It will be apparent to those skilled in the art that various modifications are conceivable to the above disclosed embodiments without departing from the scope of protection determined by the appended claims.

### Reference list

**10** dental self-tapping implant, alternatively called dental implant
**L** length of the dental implant
   **N** neck portion
      **d₁** diameter of the neck portion
   **S** proximal-central straight portion
   **C** distal conical portion
   **h₁** segment of the implant corresponding to the cortical portion of the jawbone, next to the proximal neck portion N, in short cortical segment
   **h₂** segment of the implant corresponding to the spongiosa portion of the jawbone, in short spongiosa segment
**12** body of the implant
   **t** longitudinal body axis
   **d₂** body diameter
      **d₂₋₁, d₂₋₂, d₂₋₃** body diameters for the examples of realization **d₂₋₁, d₂₋₂, d₂₋₃** where **d₂₋₁** < **d₂₋₂** < **d₂₋₃**
   **14** core
   **16** thread
   **20** flute having a circular arc shape
      **20a** longitudinal flute axis
      **r₁** a first radius along the cortical segment **h₁**,
      **r₂** a second radius along the spongiosa segment **h₂**
      **g₁** a first depth along the cortical segment **h₁**
      **g₂** a second depth along the spongiosa segment **h₂**
      **22a, 22b** cutting edges along the cortical segment **h₁**,
      **23a, 23b** cutting edges along the spongiosa segment **h₂**
   **α₁** first angle of the flute along the cortical segment
      **α ₁₋₁, α ₁₋₂, α ₁₋₃** first angle of the flute for the examples of realization, where
      **α ₁₋₁**< **α ₁₋₂**< **α ₁₋₃**
   **α₂** second angle of flute along the spongiosa segment **h₂**
      **α ₂₋₁, α ₂₋₂, α ₂₋₃** first angle of the flute for the examples of realization where
      **α ₂₋₁ < α ₂₋₂< α ₂₋₃**
   **q** first ratio **q = α₁** / **α₂**
   **k** proportionality parameter
100 multi-step drill
   **dtᵢ** drill diameter **dtᵢ**, where i= 1,2,3,4
   dt₁₋₁ dt₁-₂; dt₁-₃; dₜ₁-₄; dt₂₋₁; dt₂₋₂; dt₂-₃; dt₂-₄; dt₃₋₁; dt₃₋₂; dt₃-₃; dt₃-₄ drill diameters for the examples of realization
   **htᵢ** height of the steps of the multi-step drill, where i= 1,2,3,4

## Claims

1. A dental self-tapping implant (10) comprising a body (12) having a longitudinal body axis **(t)** and a length (L), the body (12) having a proximal neck portion (N), a proximal-central straight portion (S) of substantially constant body diameter (**d₂**) and a distal conical portion (C) of decreasing diameter, the body (12) comprising a core (14) with a thread (16) provided thereon, and a flute (20) of circular arc shape formed in the thread (16), the flute (20) runs helicoidally around the core (14), the flute (20) having a longitudinal flute axis (20a),
wherein:
the body (12) has two segments:
a cortical segment (**h₁**) next to the proximal neck portion (N),
a spongiosa segment (**h₂**) extending the cortical segment (**h₁**), the spongiosa segment (**h₂**) including the distal conical portion (C),
the sum of the cortical segment **h₁**) and the spongiosa segment (**h₂**) being equal to the sum of the proximal-central straight portion (S) and the distal conical portion C),
the flute (20) is configured to run helicoidally around the core (14) until it reaches the neck portion (N), so that the longitudinal flute axis (20) forms a first angle (**α₁**) with the longitudinal body axis **(t)** along the cortical segment (**h₁**, and forms a second angle (**α₂**) with the longitudinal body axis **(t)** along the spongiosa segment (**h₂**),
**characterized in that**:
the first angle (**α₁**) is larger than the second angle (**α₂**),
and **in that**:
the value of the first angle (**α₁**) is directly proportional to the body diameter (**d₂**) with a proportionality parameter **(k)** within the interval 11 to 18, preferably within the interval 13 to 15.

2. The dental self-tapping implant according to claim 1,
**wherein**
a first ratio (**q** is defined between the first angle (**α₁**) and the second angle (**α₂**), the first ratio **(q)** being in the interval 1.09 to 1.15, preferably 1.115 to 1.125, more preferably 1.12.

3. The dental self-tapping implant according to claim 1 or 2,
**wherein**
the flute (20) has a first depth (**g₁**) along the cortical segment (**h₁**), and a second depth (**g₂**) along the spongiosa segment (**h₂**),
the first depth (**g₁**) is smaller than the second depth (**g₂**) and
the first depth (**g₁**) and the second depth (**g₂**) increase with the body diameter d₂.

4. The dental self-tapping implant according to any of the claim 1 to 3,
**wherein**
the circular arc shape of the flute (20) has a first radius (**r₁**) along the cortical segment (**h₁**), and a second radius (**r₂**) along the spongiosa segment (**h₂**),
and **wherein**
the first radius (**r₁**) is larger than the second radius (**r₂**).

5. A set comprising a dental self-tapping implant (10) according to any of the claims 1 to 4, and a **multi-step drill (100)** configured for the dental self-tapping implant (10),
**characterized in that:**
the multi-step drill (100) has four steps along the spongiosa segment (**h₂**, each step having a drill diameter (**dtᵢ** and a step height (**htᵢ**), where **i=** 1,2,3,4,
the drill diameters **dtᵢ** increase from the portion of the multi-step drill (100) corresponding to the distal conical portion (C) to the proximal-central straight portion (S),
**and in that:**
the body diameter (**d₂**) is greater than the drill diameters (**dtᵢ**) at each step height (**htᵢ**), where **i=** 1,2,3,4, and **in that**:
the step heights (**htᵢ**) along the spongiosa segment (**h₂**) have respective predetermined values irrespective of the body diameter (**d₂**),
**and in that:**
the drill diameters (**dtᵢ**) increase with the respective body diameter (**d₂**).

6. A collection comprising at least three sets of claim 5,
**characterized in that**
the diameter (**d₂**) of each of the at least three dental self-tapping implants (10) is selected from a list of diameters that forms an increasing series with a step interval of at least 0.2 mm, preferably with a step interval of 0.3 to 0.5 mm, more preferably with a step interval of 0.4 mm, and wherein the cores (14) of the dental self-tapping implants are substantially identical for all the selected diameters (**d₂**) from the list.

## Patentansprüche

1. Selbstbohrendes Dentalimplantat (10), umfassend einen Körper (12), der eine Längskörperachse (t) und eine Länge (L) aufweist, wobei der Körper (12) einen proximalen Halsabschnitt (N), einen proximal-zentralen geraden Abschnitt (S) mit einem im Wesentlichen konstanten Körperdurchmesser (d₂) und einen distalen konischen Abschnitt (C) mit abnehmendem Durchmesser aufweist, wobei der Körper (12) einen Kern (14) mit einem daran bereitgestellten Gewinde (16) und eine Rille (20) mit einer Kreisbogenform, die in dem Gewinde (16) gebildet ist, umfasst, wobei die Rille (20) schraubenförmig um den Kern (14) herum verläuft, wobei die Rille (20) eine Längsrillenachse (20a) aufweist,
wobei:
der Körper (12) zwei Segmente aufweist:
ein kortikales Segment (h₁) neben dem proximalen Halsabschnitt (N),
ein Spongiosa-Segment (h₂), welches das kortikale Segment (h₁) verlängert, wobei das Spongiosa-Segment (h₂) den distalen konischen Abschnitt (C) umfasst,
die Summe des kortikalen Segments (h₁) und des Spongiosa-Segments (h₂) gleich der Summe des proximal-zentralen geraden Abschnitts (S) und des distalen konischen Abschnitts (C) ist,
die Rille (20) dazu konfiguriert ist, schraubenförmig um den Kern (14) herum zu verlaufen, bis sie den Halsabschnitt (N) erreicht, so dass die Längsrillenachse (20) einen ersten Winkel (α₁) mit der Längskörperachse (t) entlang des kortikalen Segments (h₁) bildet und einen zweiten Winkel (α₂) mit der Längskörperachse (t) entlang des Spongiosa-Segments (h₂) bildet,
**dadurch gekennzeichnet, dass**:
der erste Winkel (α₁) größer als der zweite Winkel (α₂) ist, und dass:
der Wert des ersten Winkels (α₁) zu dem Körperdurchmesser (d₂) direkt proportional ist mit einem Proportionalitätsparameter (k) innerhalb des Intervalls von 11 bis 18, bevorzugt innerhalb des Intervalls von 13 bis 15.

2. Selbstbohrendes Dentalimplantat nach Anspruch 1, wobei ein erstes Verhältnis (q) zwischen dem ersten Winkel (α₁) und dem zweiten Winkel (α₂) definiert ist, wobei das erste Verhältnis (q) in dem Intervall von 1,09 bis 1,15, bevorzugt von 1,115 bis 1,125, weiter bevorzugt bei 1,12 liegt.

3. Selbstbohrendes Dentalimplantat nach Anspruch 1 oder 2, wobei die Rille (20) eine erste Tiefe (g₁) entlang des kortikalen Segments (h₁) und eine zweite Tiefe (g₂) entlang des Spongiosa-Segments (h₂) aufweist,
die erste Tiefe (g₁) kleiner als die zweite Tiefe (g₂) ist, und
die erste Tiefe (g₁) und die zweite Tiefe (g₂) mit dem Körperdurchmesser d₂ zunehmen.

4. Selbstbohrendes Dentalimplantat nach einem der Ansprüche 1 bis 3, wobei
die Kreisbogenform der Rille (20) einen ersten Radius (r₁) entlang des kortikalen Segments (h₁) und einen zweiten Radius (r₂) entlang des Spongiosa-Segments (h₂) aufweist,
und wobei der erste Radius (r₁) größer als der zweite Radius (r₂) ist.

5. Garnitur, umfassend ein selbstbohrendes Dentalimplantat (10) nach einem der Ansprüche 1 bis 4, und einen Mehrstufenbohrer (100), der für das selbstbohrende Dentalimplantat (10) konfiguriert ist,
**dadurch gekennzeichnet, dass**:
der Mehrstufenbohrer (100) vier Stufen entlang des Spongiosa-Segments (h₂) aufweist, wobei jede Stufe einen Bohrdurchmesser (dtᵢ) und eine Stufenhöhe (htᵢ) aufweist, wobei i = 1, 2, 3 4,
die Bohrdurchmesser (dtᵢ) von dem Abschnitt des Mehrstufenbohrers (100) aus, der dem distalen konischen Abschnitt (C) entspricht, bis zu dem proximal-zentralen geraden Abschnitt (S) zunehmen,
und dass:
der Körperdurchmesser (d₂) größer als die Bohrdurchmesser (dtᵢ) auf jeder Stufenhöhe (htᵢ) ist, wobei i = 1, 2, 3, 4, und dass:
die Stufenhöhen (htᵢ) entlang des Spongiosa-Segments (h₂) jeweilige vorbestimmte Werte unabhängig von dem Körperdurchmesser (d₂) aufweisen,
und dass:
die Bohrdurchmesser (dtᵢ) mit dem jeweiligen Körperdurchmesser (d₂) zunehmen.

6. Kollektion, umfassend mindestens drei Garnituren nach Anspruch 5,
**dadurch gekennzeichnet, dass** der Durchmesser (d₂) jedes der mindestens drei selbstbohrenden Dentalimplantate (10) aus einer Liste von Durchmessern ausgewählt wird, die eine zunehmende Reihe mit einem Stufenintervall von mindestens 0,2 mm, bevorzugt mit einem Stufenintervall von 0,3 bis 0,5 mm, weiter bevorzugt mit einem Stufenintervall von 0,4 mm bildet, und wobei die Kerne (14) der selbstbohrenden Dentalimplantate für alle ausgewählten Durchmesser (d₂) aus der Liste im Wesentlichen identisch sind.

## Revendications

1. Implant dentaire auto-taraudant (10) comprenant un corps (12) ayant un axe de corps longitudinal **(t)** et une longueur (L), le corps (12) ayant une partie proximale de col (N), une partie droite proximale-centrale (S) de diamètre de corps essentiellement constant (**d₂**) et une partie conique distale (C) de diamètre décroissant, le corps (12) comprenant une âme (14) avec un filet (16) prévu dessus, et une goujure (20) de forme arquée circulaire formée dans le filet (16), la goujure (20) passe de manière hélicoïdale autour de l'âme (14), la goujure (20) ayant un axe de goujure longitudinal (20a),
dans lequel :
le corps (12) présente deux segments :
un segment cortical (**h₁**) à proximité de la partie proximale de col (N),
un segment de spongiosa (**h₂**) étendant le segment cortical (**h₁**), le segment de spongiosa (**h₂**) incluant la partie conique distale (C),
la somme du segment cortical (**h₁**) et du segment de spongiosa (**h₂**) étant égale à la somme de la partie droite proximale-centrale (S) et de la partie conique distale (C),
la goujure (20) est configurée pour passer de manière hélicoïdale autour de l'âme (14) jusqu'à ce qu'elle atteigne la partie de col (N), de façon à ce que l'axe de goujure longitudinal (20) forme un premier angle (**α₁**) avec l'axe longitudinal de corps **(t)** le long du segment cortical (**h₁**), et forme un second angle (**α₂**) avec l'axe longitudinal de corps **(t)** le long du segment de spongiosa (**h₂**),
**caractérisé en ce que** :
le premier angle (**α₁**) est plus grand que le second angle (**α₂**),
et **en ce que** :
la valeur du premier angle (**α₁**) est directement proportionnelle au diamètre de corps (**d₂**) avec un paramètre de proportionnalité **(k)** situé dans l'intervalle de 11 à 18, de préférence dans l'intervalle de 13 à 15.

2. Implant dentaire auto-taraudant selon la revendication 1, dans lequel
un premier rapport **(q)** est défini entre le premier angle (**α₁**) et le second angle (**α₂**), le premier rapport **(q)** étant situé dans l'intervalle entre 1,09 et 1,15, de préférence entre 1,115 et 1,125, de manière plus préférée de 1,12.

3. Implant dentaire auto-taraudant selon la revendication 1 ou 2, dans lequel,
la goujure (20) présente une première profondeur (**g₁**) le long du segment cortical (**h₁**), et une seconde profondeur (**g₂**) le long du segment de spongiosa (**h₂**) ; la première profondeur (**g₁**) est plus petite que la seconde profondeur (**g₂**) et
la première profondeur (**g₁**) et la seconde profondeur (**g₂**) augmentent avec le diamètre du corps (**d₂**).

4. Implant dentaire auto-taraudant selon l'une quelconque des revendications 1 à 3, dans lequel
la forme d'arc circulaire de la goujure (20) présente un premier rayon (**r₁**) le long du segment cortical (**h₁**), et un second rayon (**r₂**) le long du segment de spongiosa (**h₂**),
et dans lequel
le premier rayon (**r₁**) est plus grand que le second rayon (**r₂**).

5. Kit comprend un implant dentaire auto-taraudant selon l'une quelconque des revendications 1 à 4, et un **foret multi-étages (100)** configuré pour l'implant dentaire auto-taraudant (10),
**caractérisé en ce que** :
le foret multi-étages (100) présente quatre étages le long du segment de spongiosa (**h₂**), chaque étage ayant un diamètre de foret (**dtᵢ**) et une hauteur de foret (**htᵢ**), où **i** = 1, 2, 3, 4,
les diamètres de foret (**dtᵢ**) augmentent de la partie du foret multi-étages (100) correspondant à la partie conique distale (C) à la partie droite proximale-centrale (S),
et **en ce que** :
le corps de diamètre (**d₂**) est supérieur aux diamètres de foret (**dtᵢ**) à chaque hauteur d'étage (**htᵢ**) , où **i** = 1, 2, 3, 4, et **en ce que** :
les hauteurs d'étage (**htᵢ**) le long du segment de spongiosa (**h₂**) ont des valeurs prédéterminées respectives quel que soit le diamètre de corps (**d₂**),
et **en ce que** :
les diamètres de foret (**dtᵢ**) augmentent avec le diamètre de corps (**d₂**) respectif.

6. Collection comprenant au moins trois kits selon la revendication 5,
**caractérisée en ce que**
le diamètre (**d₂**) de chacun des au moins trois implants dentaires auto-taraudants (10) est sélectionné parmi une liste de diamètres qui forme une série croissante avec un intervalle d'étage d'au moins 0,2 mm, de préférence avec un intervalle d'étage de 0,3 à 0,5 mm, de manière plus préférée avec un intervalle d'étage de 0,4 mm, et dans lequel les âmes (14) des implants dentaires auto-taraudants sont essentiellement identiques pour tous les diamètres sélectionnés (**d₂**) de la liste.
